# EUROPEAN PATENT APPLICATION

(11) **EP 2 996 058 A1**
(43) Date of publication of application: **16.03.2016**
(21) Application number: 14306396.4
(22) Date of filing: 10.09.2014
(51) Int. Cl.: G06F 19/00

(54) **Method for automatically generating representations of imaging data and interactive visual imaging reports**

(71) Applicant: Intrasense, 34000 Montpellier (FR); Gallix, Benoit, Montreal, Québec H4A 3M1 (CA)
(72) Inventor: Gallix, Benoît, Montreal, Québec H4A 3M1 (CA); Feldmar, Jacques, 94160 Saint Mande (FR); Banegas, Frédéric, 34070 Montpellier (FR)
(74) Representative: Pontet Allano & Associes

(57) **Abstract**

A method for automatically generating a mode of presentation for data collected and produced during an imaging examination for which an examination report is done by an operator in charge of said examination, comprising:
- analyzing contextual information related to said examination,
- analyzing data contained in said examination report,
- eliciting and producing relevant information from and within said collected and produced data, based on results of said contextual information analysis and of said report data analysis, and
- displaying said relevant information, in a simplified multi-dimensional manner as an interactive visual imaging report.

Contextual information comprise information on the behavior of the operator while achieving and reporting said examination.

Interactive Visual Imaging Reports (IVIR) are automatically generated, as computed multi-dimensional and multi-scale objects, from native images and information collected during the image acquisition and interpretation process.

## Description

The invention relates to a method for automatically generating representations of data obtained from imaging systems. This applies to the medical imaging field as to other areas of imaging technology and pertains to the creation of Interactive Visual Imaging Reports (IVIRs) generated by this method.

### BACKGROUND

Techniques implemented for acquiring radiologic images are increasingly complex with the interpretation of said images becoming ever more difficult. In medicine, two specialties specifically use image analysis to arrive at a diagnosis: diagnostic medical imaging and pathology both requiring dedicated and distinct skillsets and training. However, while pathology communication to clinicians is now predominantly based on the access to a text report including conclusions, radiology has long been conventionally structured around the association of a report and the 'radiological picture' of organs which could be viewed and simultaneously interpreted by physicians.

Nowadays, the sheer quantity and complexity of images provided by new imaging techniques result in tremendous difficulty for a non-radiologist to easily view and understand such images. Moreover, new imaging modalities such as scanners, MRIs, PET scans or nuclear medicine techniques, produce a large amount of data which is transmitted from imaging centers to physician offices most often in the form of CD-rom. These datasets are generally provided unprocessed and are difficult to be visualized without specialized dedicated software. A physician, in front of his or her patient, is faced with difficulties providing a clinically relevant interpretation and diagnosis from non-interpretable imaging data and consequently is no longer in a position to monitor his or her patient's pathology and deliver an ideal treatment and prognosis. Clinical decision making is done on the basis of the radiologist textual report only and less and less in the context of the actual radiologic image themselves which have become too complex.

For these reasons, non-medical imaging professionals are progressively losing their ability to read and understand radiologic images and rely only on written reports and conclusions produced by the radiologist. Key image selection and workstation post-processing is sometimes performed by the radiologist for visual annotation of the textual report. However this is very time consuming and may not be consistently reproducible. In routine practice, visual annotation and manual manipulation of the imaging dataset is not sustainable.

WO2010109351A1discloses a system that automatically retrieves report templates based on diagnostic information. When generating radiology reports, image findings and/or clinical information is automatically mapped to an appropriate standardized structured report template. The report template contains placeholders for information such as case-specific images and measurable values, and the placeholders are filled in by either the radiologist or by automatic procedures such as image processing algorithms, text extraction algorithms, etc. In this manner, the radiologist is assisted in effectively generating a reader-independent high-quality diagnostic report.

WO2008057229 A2discloses a custom report generation system for medical information, comprising: determining key medical images and medical reports; determining a clinician's preferences for medical records obtained from a physician; determining the clinician's preferences for clinical information system records; determining the clinician's preferences for display of the medical images, medical reports, medical records, and clinical information system records; and displaying the medical images, medical reports, medical records, and clinical information system records.

EP2 169 577A1 discloses a method for supporting a preparation of medical report for a patient, comprising acquiring one or more medical imaging studies and/or medical records which are related to the patient, automatically matching a report template to the one or more medical imaging studies and/or medical records according to at least one characteristic thereof, presenting the matched report template to allow a user to provide a diagnosis of the one or more medical imaging studies and/or medical records, and embedding the diagnosis in the matched report template.

These report generation systems are mainly based on the manual annotations input by a radiologist and are not really adapted for a flexible use by physicians.

The objective of the invention is to provide a method for automatically producing, without any active contribution of radiologists, a way for displaying medical imaging data on a pad, tablet, smartphone, computer, or other electronic display device, that would allow any medical doctor to review in a simplified manner, complex imaging examinations.

### SUMMARY

This objective is reached with a method for automatically generating a mode of presentation for data collected and produced during an imaging examination for which an examination report is done by an operator in charge of said examination, comprising:
- analyzing contextual information related to said examination,
- analyzing data contained in said examination report,
- eliciting and producing relevant information from and within said collected and produced data, based on results of said contextual information analysis and of said report data analysis, and
- displaying said selected relevant information, in a simplified multi-dimensional manner as an interactive visual imaging report,
wherein said contextual information comprise information on the behavior of said operator while achieving and reporting said examination.

In this way, a physician can access an interactive visual imaging rendering that can be displayed on a tablet, smartphone or computer. The interactive capacity of this rendering allows the physician to adapt the rendering to his skill level and to his or her questions about their patient's pathology. According to another aspect of the invention, an interactive visual imaging report (IVIR) is proposed, in relation with a medical examination achieved on a patient, said IVIR comprising:
- a global multidimensional view of said patient, including graphic information pointing one or multiple organ of interest concerned by said examination, and
- a superimposed multidimensional view that highlight the findings detected during the process of reporting the examination.

This interactive visual imaging report (IVIR) according to the invention may further comprise a plurality of interactive visual imaging report (IVIR) levels for displaying images, data extracted from images or schematics of the organ of interest, said interactive visual imaging report being provided with a multi-scale functionality for moving from one of said IVIR levels to another IVIR level.

The IVIR according to the invention may also comprise a multidimensional rendering of the organ of interest, said multidimensional rendering being accessed by rotating and/or translating and/or zooming on said organ.

**An imaging examination** can be referred to as a technique and process of creating and analysing understandable image in order to capture information that is not directly accessible to human vision. A medical imaging examination creates and analyzes visual representations of the interior of the human body. Other imaging examinations include industrial imaging, satellite imagery, radar imagery, airport security imaging, sonar imaging, or microscopic imaging. The imaging examination makes it possible to produce an examination report. The period of time associated with the imaging examination lasts from the arrival of the patient and the requisition in the radiology department to the production of the examination report and its communication to the referring physician and other relevant care providers.

**An examination report** can be defined as the result of interpreting the set of image data created during an imaging examination. The examination report is a written account that describes the findings and/or impression and/or diagnosis and/or abnormality that has been observed or detected when analyzing the data set. When a physician or a clinician requests a radiologic examination for a patient, said patient goes to a medical imaging center which has access to their previous radiologic examinations and electronic medical file.

At the imaging center, a radiologist reads the requisition issued by the physician and may converse with the patient and other clinicians. He or she gives instructions of an acquisition protocol to a technologist. Said technologist acquires series of views and may perform image post-processing, measurements, and produce key-images according to the protocol.

The radiologist retrieves the series on the post-processing station, and other information provided by the technologist. He or she analyses the series occasionally in comparison to previous series. He or she can also produce objects such as annotations, areas of interest, or measurements in these series. The radiologist eventually submits a report.

**A medical image** can be referred as a volume made of voxels (element of volume with an associated value). A schematic representation of a medical image is a visual object extracted from the medical image either automatically, semi-automatically or manually or a combination there of by an operator.

Objects are conceptually of a "higher-level" than the voxels, meaning that they are easier to understand and interpret by the human brain and require less expertise and effort to be understood. Objects summarize a set of voxels (for example an organ of interest or a region of interest).

**A schematic representation** is usually 2D or 3D and made of lines or surfaces, with color coding making easy interpretation and understanding of what these objects are. A schematic representation can easily be displayed and selected by a click on the element.

**A schematic rendering** is a simplified and symbolic representation that illustrates the information recorded during the imaging examination and the findings observed during the reading process.

**An operator** refers to the person (or group of person) who operates the equipment that produces an imaging examination.

**A reader** is a person (or a group of persons) that reviews and inspects the image dataset, uses specific tools to analyze the image contents, and creates the examination report in order to record the findings in a written text. Note: Operator and reader may or may not be distinct persons.

**IVIR or Display level** refers to the possibility to adapt the complexity of the information and the volume of the data to the type of user and the performance of the device used to display the image.

**Imaging examination ordering** refers to the written text that is used to request an imaging examination to be made. It explains the reasons for carrying out an imaging examination, and contains information on the circumstances that could be helpful to better understand and analyse the imaging examination.

**Contextual information** can be defined as all the information which is accessed, retrieved, collected, and produced during the imaging acquisition and interpretation. The contextual information may comprise information on the clinical context of the examination request. Said information on the context of the examination may request comprise information extracted from a patient's medical file.

The contextual information may comprise information on the process of creation and/or interpretation of the examination. Said information on the process may also comprise information on the protocol chosen for the examination.

A **region of interest (ROI)** is a selected subset of samples within an image dataset identified for a particular purpose: on an image (2D dataset), the boundaries of an object, in a volume (3D dataset), the contours or surfaces outlining an object (Volume of Interest (VOI)) and in a time-volume (4D dataset), the outline of an object at or during a particular time interval. A region of interest may comprise a subset of voxels which is relevant for diagnosis. It is usually delineated by a surface which can easily be rendered. The ROI can be produced automatically, semi-automatically or manually.

A **point of interest** is a location in the image volume which is relevant for diagnosis. It is usually represented by an icon or an annotation which can easily be rendered. The POI can be produced automatically, semi-automatically or manually.

An **organ of interest** is a volume of interest within a 3D data set or a specific volume during a specific time (4D) that represents a specific organ that is typically self-contained and has a specific function, such as the heart, lung, or liver in the human body.

A **point or an area of interest** refers to a specific zone of an image dataset (2D or 3D) or a specific zone during a specific time interval (4D) which has been the subject of special attention during the reading process or has been identified by the reader of specific interest for the analysis of the dataset.

The interactive 3D schematic rendering may comprise a three-dimensional rendering of anatomic structures that can be displayed or rendered with low computing resources.

The interactive 3D schematic rendering may comprise a three-dimensional rendering of relevant information that is superimposed on reference organs.

The interactive 3D schematic rendering may comprise a highlighted display of relevant information on one or more three-dimensional anatomic objects of interest within said rendering.

The interactive 3D schematic rendering may comprise displaying information that was acquired during the examination, with said displayed information being superimposed on a three-dimensional anatomic object in relation to said information.

A **structure** (or data structure) is a particular way of organizing data in a computer so that it can be used efficiently by algorithms. In the context of this invention, structures are typically trees, graphs or lattices which are linked abstract data structures composed of nodes.

A **node** is an element of a structure containing data and one or more pointers to other nodes.

Contextual information about the exam is analyzed to determine a suitable rendering process and representation to generate the multi-dimensional rendering. The method of invention is further comprised of a sequence for creating an interactive visual imaging report (IVIR) including the following steps:
- creating structured data from information collected during a patient's protocoled examination track,
- merging independent or correlated data,
- determining a format for said IVIR, which corresponds to the protocoled examination,
- automatically generating secondary data if necessary,
- associating said structured or generated data with the interactive visual imaging report structure.

The interactive visual imaging report may also include:
- a global three-dimensional view of said patient, including graphic information pointing an organ of interest concerned by said examination, and
- contextual information related to said examination.

The interactive visual imaging report further comprises a multi-dimensional rendering of the organ of interest, said multi-dimensional rendering being accessed by zooming in on said organ.

The automatic report-creation method according to the invention is distinct from the prior art in that the proposed interactive visual imaging reports are better suited to the needs of users according to their skills.

The automatic generation starts only after the end of the interpretation of the images, based on available information before, during or after the report production process. The radiologists are not active or conscious contributors to the report generation process which is derived from a plurality of data from various origins. The report generated by the method according to the invention may be variable in length and complexity (multi-tiered presentation styles), so as to be suited to the reviewing user's skills and display device.

It is important to note that the method according to the invention is independent of any individual presentation device.

As an IVIR is interactive, the objects displayed on each screen is dynamic and can enable the user to switch from one representation to another. Different representations show different levels of details. An IVIR contains multiple screens. On each screen, objects such as surfaces, sections, regions of interest (ROI), annotations of interest (AOI) are displayed or hidden, and each object can be clicked to allow navigation from one screen to another. The screens present multiple levels-of-detail which determines their IVIR level.

It has to be noted that an object can be enabled or hidden without changing the level-of-detail. Different structures of data can be used for different IVIRs. Such structures can be automatically determined among a finite set of structures predefined by Experts.

Different types of rendering suited for various display formats and mobile supports (browsers, tablets, smartphones, etc.) can be implemented by means of IVIRs according to the invention. The role of rendering is to transform an IVIR into screens. A screen is associated to each node, displays the objects associated to this node. It also enables the user to interact with the object and to move to other screens displaying other nodes of the IVIR.

The template of an IVIR contains nodes, each node being associated to an IVIR level. The lower the IVIR level, the closer to the raw data (image, voxel). The higher the IVIR level, the closer to a fully schematic and symbolic representation. The high levels will tend to have a lower resolution than the low levels.

There is an implicit relationship between the IVIR level and the complexity of the objects which are displayed at this level. The low levels contain more details while the high levels contain more symbolic and schematic information. The weight of the data associated to low levels will tend to be larger than those associated to high levels meaning that summarization and data compression occurs when switching from lower to higher levels.

This intelligent summarization makes the invention adaptive to the expertise of the person watching the IVIR level and to the information he/she needs at a given time in the IVIR visualization. One could claim that the data compression is semantically lossless since the information which is lost from the low to high level is not clinically relevant to the recipient.

### BRIEF DESCRIPTION OF THE DRAWINGS

- **FIG.1** features a first level for an interactive visual imaging report (IVIR) created by the method according to the invention;
- **FIG.2** features the 3D rendering of the organ of interest (liver) and area of interest (liver tumor, FIG.1, whereas others body organs (e.g. lung, bones, cardiovascular system, ...) have been hidden;
- **FIG.3** features the 3D rendering of the liver (organ of interest) wherein internal vasculature is highlighted;
- **FIG.4** features the 3D rendering of FIG.3, wherein the user has selected to view only the liver surface, hepatic vein, and tumor;
- **FIG.5** illustrates that by clicking on the axial icon the readers can easily review the native 2D axial images of the liver, and keep the tumor highlighted in purple for better visualization, as well as featuring a second level for an IVIR, displaying 2D sections of the organ subject from the examination;
- **FIG.6** features multi-planar axial and coronal views of a liver, obtained from the 2^{nd} level of the IVIR featured in FIG.5;
- **FIG.7** features a measuring step in the axial view of FIG.6;
- **FIG.8** features a third level of an IVIR, wherein a plurality of series are displayed;
- **FIG.9** features a selection of a set displaying a tumor, from the third level featured in FIG.8;
- **FIG.10** features a full display of the tumor selected in FIG.9;
- **FIG.11** illustrates the multi-factorial contextual inputs (i.e. volume of image data, display device, user knowledge) of IVIRs according to the invention;
- **FIG.12** illustrates a standard workflow of an examination, leading to the automatic generation of an IVIR according to the invention;
- **FIG.13** illustrates a step for making a series of imaging sections within an IVIR according to the invention;
- **FIG.14** illustrates various levels of imaging representations within an IVIR according to the invention;
- **FIG.15** illustrates a specific embodiment of a method for automated IVIR generation according to the invention;
- **FIG.16** is a schematic view of a free-text analysis module implemented for providing structured information from data collected during the examination;
- **FIG.17** is a schematic view of a header analysis module implemented for providing structured data from dataset images and headers;
- **FIG.18** is a schematic view of an image-analysis and recognition module implemented for providing structured data from input images data collected before, during and following the current examination;
- **FIG.19** is a schematic view of a module for analyzing a radiologist's behavior, implemented in the automated IVIR generation method according to the invention;
- **FIG.20** illustrates a step for merging structured data within the automated IVIR generation method according to the invention; and
- **FIG.21** illustrates an instantiation process implemented in the automated IVIR generation method according to the invention.

### DETAILED DESCRIPTION

An Interactive Visual Imaging Report (IVIR) produced according to the invention is composed of a mixture of 2D, 3D interactive or 3D enhanced renderings (possibly 3D + time) of a human body or of its main organs, corresponding to an area of interest explored during an imaging examination. By means of such a rendering, a user can intuitive correlate the report with radiologic observations: normal or abnormal anatomy. An IVIR is a computed multidimensional and multiscale object, which is generated from native images and information collected during the image acquisition and interpretation process, from the requisition of the examination to the production of the examination report. Any useful information that is available in the patient's medical file can also be processed.

The IVIR features the main anatomic structures that have been explored during an examination. For example, these structures may include: the skeleton, the lungs, the brain (grey matter, white matter, ventricles), the face, the liver, the kidneys and the urogenital system, lymph nodes, spleen, muscles, heart and vessels.

The anatomic structures that are computationally segmented may be easily accessible via a 3D rendering through a lightweight computer device, which allows for the utilization of display tools such as tablets or smartphones. Other information contained in native images that is not automatically segmented, can be displayed by using other 3D or multidimensional rendering means which could be superimposed over reference organs.

Lesions and discovered abnormalities, which could be useful to the understanding of the patient's pathology could be highlighted on the 3D object by means of a segmentation or a specific display mode. Alternatively, the lesion may also be manually segmented during the examination interpretation or image post-processing stages.

So-called functional information, such as nuclear medicine trackers, functional sequences for MRI systems, may be superimposed over the 3D anatomic object.

A physician can access an IVIR according to the invention from:
- a paper report (e.g. smartphone Bar/QR code reading),
- a computer report,
- direct Web access to the IVIR.

Multi-dimensional - 2D, 3D or 4D objects are accessible to physicians within an IVIR, through a tablet, smartphone or computer display, which allows:
- displaying anatomic segments which have been explored during the examination,
- rotating said 3D object according to the standard six-axes,
- zooming the 2D or 3D object or modifying the centering of said object,
- interacting with anatomic structures or organs, and with lesions that have been discovered (provided that said structures, organs and lesions have been segmented);
- intuitively adding or cancelling 2D or 3D information from the 2D or 3D object;
- displaying, in an optimized manner, native or post-processed images of an anatomic area, of an organ, or a lesion, or any structure that has been initially segmented, triggered by a click from the 2D or 3D object;
- displaying previous key-images previously marked by a radiologist for the same patient, on the 2D or 3D object, in order to make historical comparisons.

The method according to the invention may provide multi-modal and patient follow-up as images obtained by other imaging techniques (for example MR, CT, multiphase, multi-parametric images) can be included in the IVIR.

An action on the anatomic areas, on the organs, the lesions or on any beforehand segmented structure can result in displaying textual information relating to:
- size, density, signal, etc.
- automatic detection and interpretation of contracted language in the conclusion of the radiologist's report,
- accessing native images with an optimized lesion display (Multiplan, and/or multiphase, and/or multi-technical, and/or comparative).

The segmented objects can be easily handled through a simple interface. For example, a click on a segmented area can make this area disappear, with the object becoming accessible in the form of a side icon, and a click on said icon reintegrates said object within the main image.

There are many possible IVIR structures. One of the tasks implemented in the method according to the invention is to identify the appropriate IVIR structures. An IVIR structure can have different numbers of levels [see definitions of "IVIR" and IVIR levels". The following example has 4 levels but IVIR structures can have more or less levels.

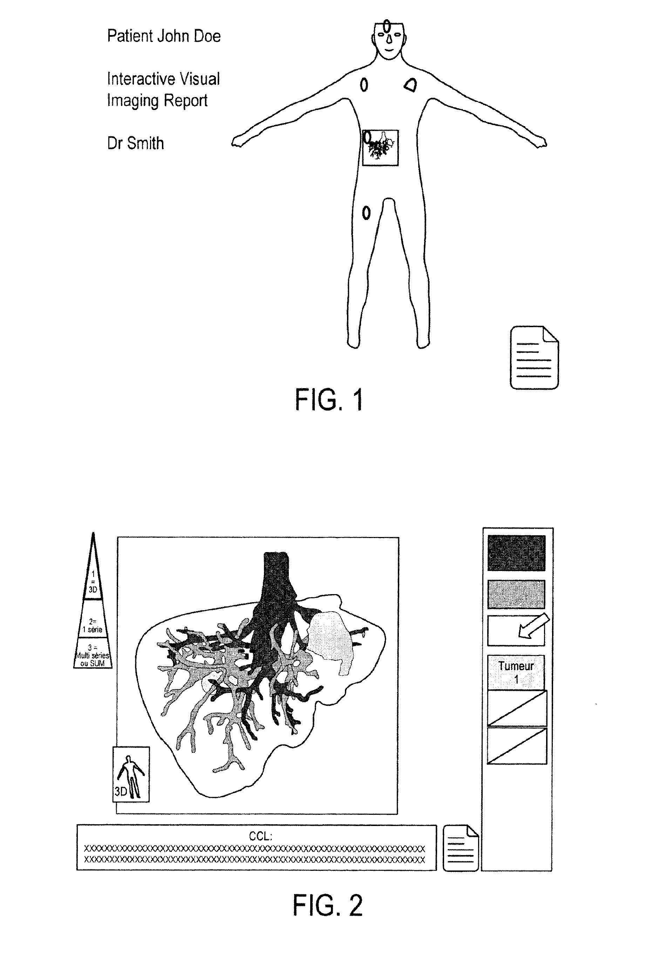

With reference to **FIG.1****,** a superior level (Level 0) of an IVIR includes a global 3D view of a patient. This view shows that the requested examination relates to the patient's liver. By clicking on this element/organ, a physician may access a 3D view of the patient's liver.

The 3D image immediately demonstrates the anatomic body region that is being explored (as depicted in the figure, a thorax, abdomen and pelvis), but also the organ and area of interest consistent with the findings observed by the reader (liver and liver tumor in this example). This 3D image is interactive and can be manipulated (i.e. axis rotation, translation, zoom). This image is consists of numerous organs separately segmented and colored (in this example, bone= white, lung=yellow, cardiovascular = red, liver = blue). Each organ can be selected to allow the reader to focus his attention on the desired organ and pathology. The findings observed by the reader (e.g. tumor in the liver) are highlighted, by clicking on the colored 3D organ. This object is then displayed separately in a new window.

Referring to **FIG.2****,** the level 1 of the IVIR shows a 3D synthesis of the requested examination on the patient's liver, including a view of the vessels and lesions. The 3D image is interactive and is composed of different structures of the organ of interest that have been automatically segmented and colored (liver surface = blue, tumor= purple). Each structure of the organ of interest can be selectively selected, highlighted or hidden, to allow the reader to focus his attention on the anatomical structure necessary.

A right column selectively displays or hides 3D elements. Matching between displayed elements and icons located in said right column is achieved by color codes (unrepresented in the Figure). 3D elements may also be selectively hidden or displayed by directly clicking on said elements. An arrow illustrates a click by the user to hide the surface of the liver.

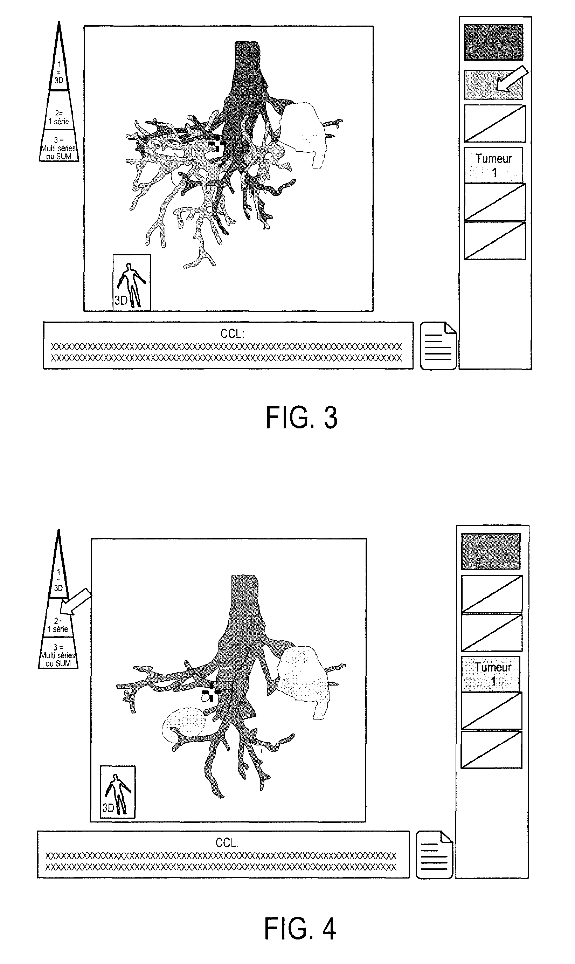

As illustrated by **FIG.3** (still level 1), the surface of the liver is now hidden and the physician decides to hide "red" vessels. Thus, he or she clicks on the icon corresponding to said "red" vessels.

This click is featured in **FIG.3** by the arrow pointing the second icon in the right column (For example: portal vein = light blue, hepatic vein = dark blue, arteries = red, surface = blue, tumor= purple). Each structure of the organ of interest can be selectively selected, highlighted or hidden, to allow the reader to focus his attention on the anatomical structure he needs.

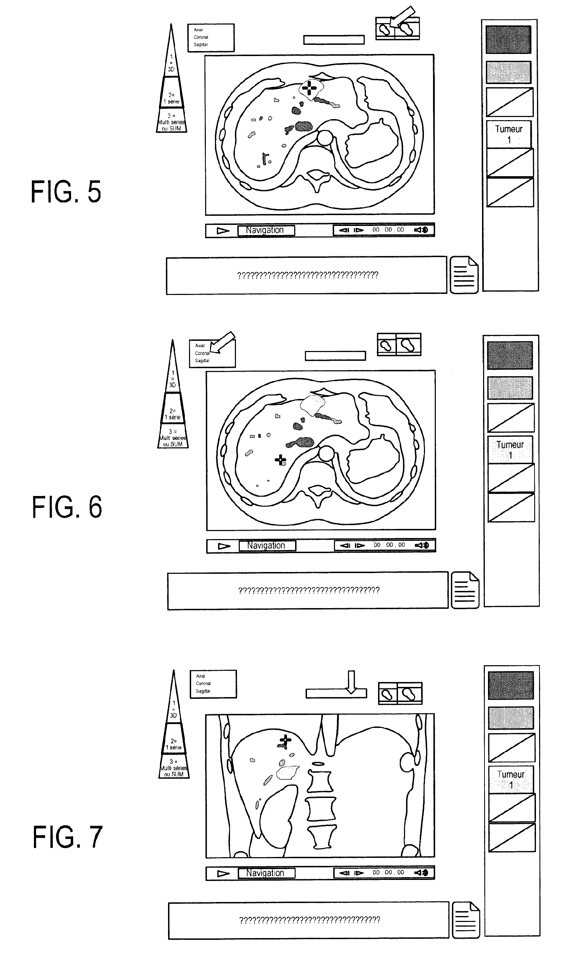

When the physician/user wants to display sections of a main set of the examination, said physician selects the level 2 on the left edge of the interface (see **FIG.4****).** This results in a display depicted by **FIG.5****.** A right column allows displaying or hiding structures. The structures that were visible in 3D at level 1 are now visible in these views. New control elements are displayed:
- at the top left, axial, coronal or sagittal views can be chosen,
- at the top middle, a rule permitting the activation of a measuring tool in the image,
- at the top right, 2D display modes for areas of interest can be selected.

The user can navigate within the 2D views. In **FIG.5****,** the arrow points to an icon provided for selecting a specific mode wherein only the contours of the structures of interest are displayed.

As illustrated by **FIG.6****,** the interface of the IVIR includes an icon (indicated by an arrow), provided for selecting coronal views of the targeted structure, here the liver.

If the physician/user wants to get a measure in the coronal view, he or she then clicks on a "rule" icon located on the top middle of the interface, with reference to **FIG.7****.**

This "measure" selection results in an interface where the physician can measure the maximum diameter of the lesion, and then clicks on a "sagittal" selection.

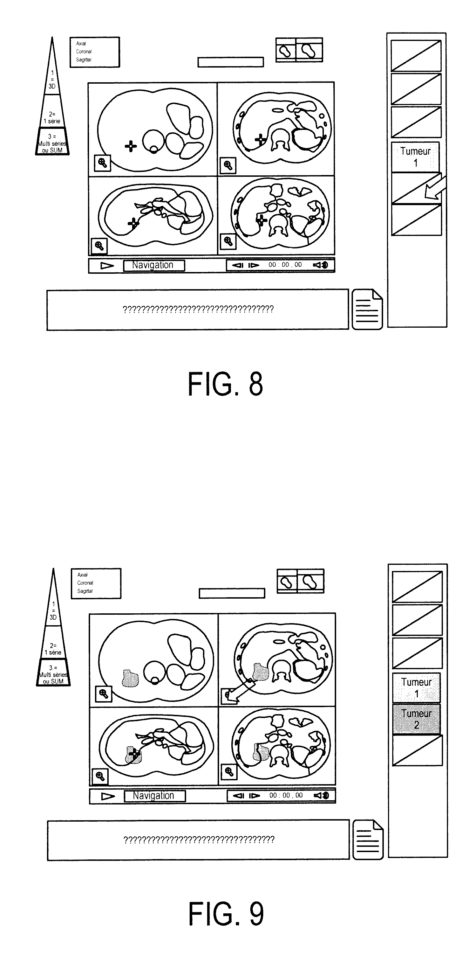

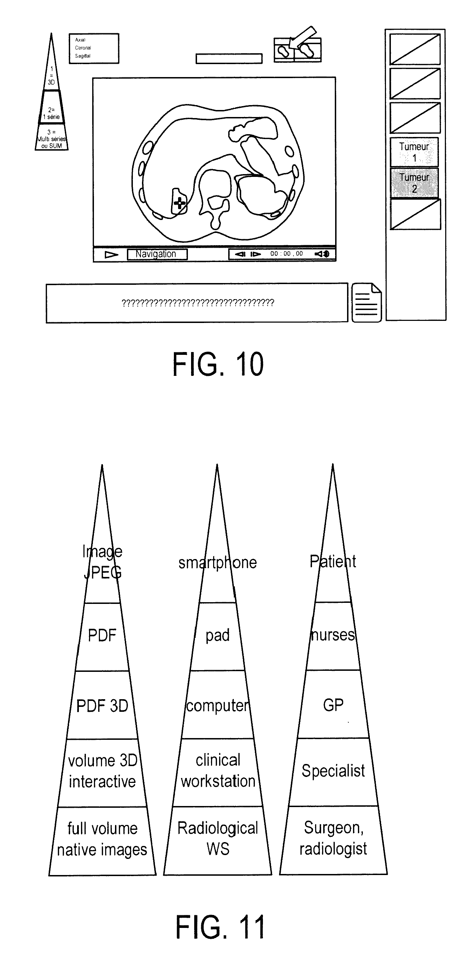

This selection results in a new sagittal view. The user can then select a mode for displaying series of views by activating the Level 3. At this level, illustrated by **FIG.8****,** the IVIR displays several series which are synchronized. The available tools are similar to those of Level 2. The user can navigate within each set, change the orientation and make measures. **FIG.8** illustrates a selection of "Tumor 2" as pointed by the arrow. With reference to **FIG.9****,** the tumor 2 is now visible. The user wants to get a full-screen view of the set displayed on the top right quarter of the interface. He then clicks on a "magnifying glass" (see arrow), resulting in a comeback to Level 2 of the IVIR, as illustrated by **FIG.10****.** From this interface, the user can decide to commute back to Level 3 in order to simultaneously display four series of the examination, and possibly to Level 1 in order to display other structures of interest.

The generation of the Interactive Visual Imaging Report (IVIR) according to the invention is automatically achieved using the patient's timeline which extends from the requisition of the imaging examination to the production of the radiologic report. The IVIR is based on data and information generated during the patient's clinical timeline. This information source has a significant amount of variability and heterogeneity as illustrated by **FIG.11****.**

Computer tools for segmenting organs and for detecting abnormalities are implemented along with an analysis of actions of the radiologist and parameters used by him/her in order to produce interpretation of results of the examination. Information provided by the requesting clinician or issued from the patient's medical file can also be used to enhance the precision of the IVIR.

The automated report-generation method according to the invention transforms a large amount of data and information into a simple, structured and interactive representation which facilitates interpretation and clinician understanding.

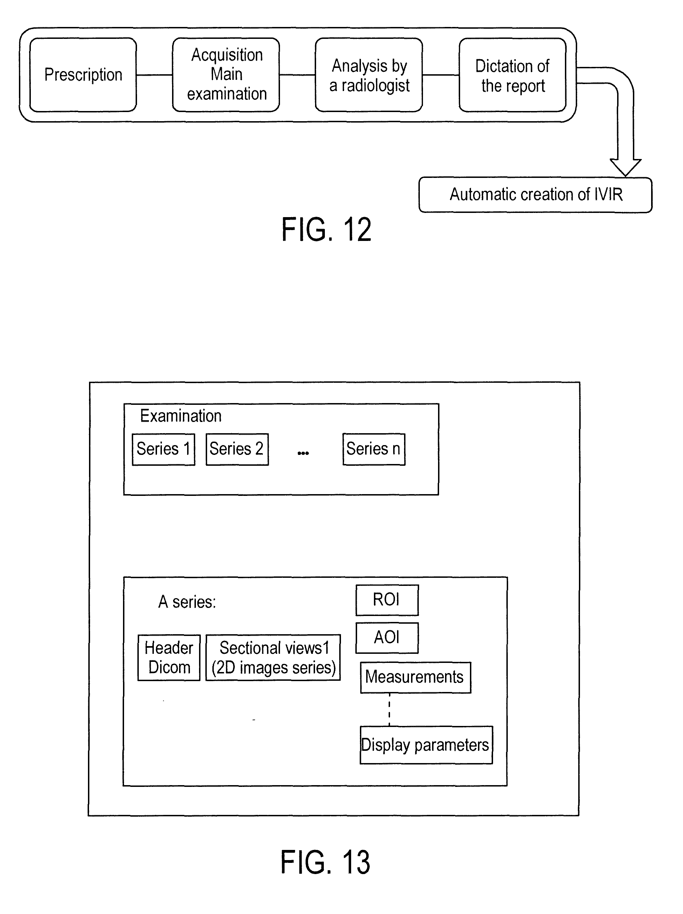

The main steps of the generation method according to the invention will now be described with reference to **FIGS 12** to **21****.**

When a physician or a clinician requests a radiologic examination for a patient, said patient goes to a medical imaging center which has access to previous radiologic examinations and to an electronic medical file for this patient **(****FIG.12****).**

At the imaging center, a radiologist reads the requisition issued by the referring physician and may discuss the clinical situation with the patient. He or she gives directs an acquisition protocol to a technologist. Said technologist acquires a series of views and may perform image post-processing, measurements, and produce key-images according to the protocol, with reference to **FIG.13****.**

The radiologist retrieves the series on the post-processing station, and other information provided by the technologist. He or she analyses the series as well as previous series. He can also produce objects such as annotations, areas of interest, and measurements in these series. The radiologist eventually finalizes a report.

From the patient's visit to the referring physician to the production of the report, information is produced by multiple individuals.

Traditionally, the referring physician's request is usually drafted as free text, with more recent order entry systems generating imaging requests via an electronic form.

At the imaging center, for the requested examination, a number of series of images are generated. DICOM headers provide in a standardized format, structured information attached to the imaging data. For example, said headers typically contain:
- the modality of the set
- the patient's name and age
- the anatomic region being imaged,
- information on the acquisition protocol.

The technologist and the radiologist may add additional information to these series, for example:
- regions of interest (ROI),
- annotations of interest (AOI),
- measurements,
- window levels for displaying the sections,
- thresholds for displaying various structures as surface renderings,
- coloring parameters for the volume rendering.

Moreover, prior examinations may be accessible through the PACS (Picture Archiving and Communications System) and the personal electronic medical record. Significant information on the patient and their past medical history is also available and can be used by the radiologist for interpreting the requested present examination.

The recording of all the performed actions by the radiologist on the present examination, along with the order and the duration of each of said actions, contains contextual information which is essential for the automated generation of the IVIR, for example:
- the displayed series of the prescribed examination and the time spent for each set,
- the selected display protocols, windows, zoom, filters and post-processing,
- the time spent viewing each section of the series and localization of the anatomic center;
- the achieved segmentations and measurements,
- the type of display,
- the consultation of historical examinations,
- the matching between the series of a same examination, or with historical examinations,
- the consultation of an atlas,
- measurements or annotations,
- level of progress in the generation of the report as a function of the viewed images,
- the use of a specific model for the report,
- the use of a computing module for assistance with image analysis,

The radiologist can access information on the patient, which is contained in a Historical Information System (HIS) or Radiology Information System (RIS) in which a review of previous examinations is stored. The report issued by the radiologist may comprise:
- free text,
- preformatted free text (template),
- a structured report,
- key images.

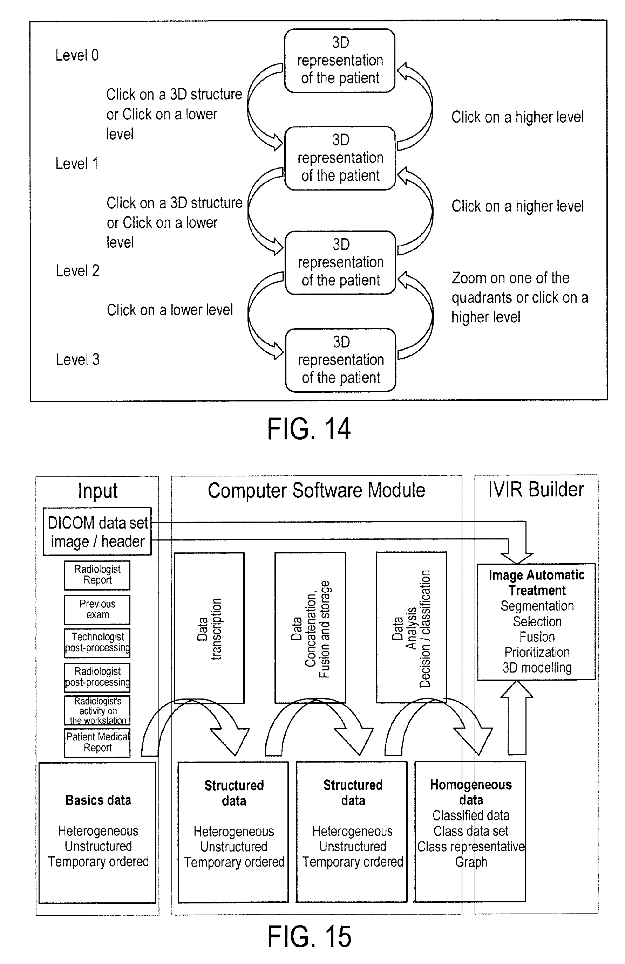

In order to automatically produce the IVIR, with reference to **FIG.15****,** it is necessary to:
- determine an appropriate structure of the IVIR among many available IVIR structures, i.e. the set of interfaces/screens which are included within the IVIR, the type of object to be displayed, the type of display for these objects, the way to navigate between the screens.
- determine the objects which are included and displayed in the IVIR for the selected structure.

An IVIR, automatically generated according to the invention, contains multiple screens. On each screen, objects such as surfaces, sections, regions of interest (ROI), annotations of interest (AOI) are displayed or hidden, and each object can be clicked to allow a navigation from one screen to another. The screens may present multiple sets of representations which will determine their IVIR level.

The structure of data, also called "template", presents the following features:
- each node contains objects,
- each node belongs to a level belonging to a set of levels illustrated by **FIG.14****,**
- actions are attached to the objects of the nodes and allow a user to go from one node to another; these are transitions between nodes.

Objects may be clicked without changing the current level-of-detail (hide/show function).

Different types of rendering suited for various display formats and mobile device support (e.g. browsers, tablets, smartphones, etc.) can be implemented by means of IVIRs. The role of rendering is to transform an IVIR into screen. One screen is associated to each node and displays the objects associated to this node by the mean of graphical rendering. It also enables the user to interact with the object and to move to other screens displaying other nodes of the IVIR.

The underlying graph structure associated to the nodes and transitions of an IVIR is typically a tree or grid since each node belongs to an IVIR level and there is a hierarchical relationship between levels.

Other data structures can be used for other IVIRs. Such structures can be automatically determined amongst a finite set of structures predefined by medical experts.

Different interactive visual imaging levels are better suited to the needs of users according to their skills. The IVIR levels and structures have a variable volume and complexity (multi-level approach), so as to be suited to the receiving user's skills and tools.

The overall information collected during the patient's clinical course is heterogeneous. In order to be able to decide upon the structure of the IVIR for a prescribed examination and objects to associate to this IVIR, it is necessary to structure all the available information.

Examples of structured data are given below:

**TABLE 1: Data relating to the requisition**

| **Type of data** | **Example of possible values** | **Example of associated element** |
|---|---|---|
| Pathology | Fracture, cancer, aneurysm | Location (ROI or Bounding Box) |
| Type of Examination | Abdomen, Thorax | |
| Modality | MRI, CT | MRI Sequence, Contrast Injection Protocol |

**TABLE 2: Data relating to the examination**

| **Type of data** | **Example of possible values** | **Example of associated element** | **Relevance** |
|---|---|---|---|
| Pathology | Fracture, cancer, aneurysm | Location (ROI or Bounding Box) | |
| Type of Examination | Abdomen, Thorax | Requested MRI sequences | |
| Set 1 | CT Thorax, MRI Prostate | MRI Sequences, Contrast Injection Protocol | Number : 1 of 10 |

**TABLE 3: Data relating to each set**

| **Type of data** | **Examples of Possible values** | **Example of Associated Element** | **Relevance** |
|---|---|---|---|
| Modality | MRI, CT | MRI Sequences, Contrast Injection Protocol | Number : 1 of 10 |
| Anatomic Region | Thorax, | Lower limbs | |
| Abnormality 1 | Lesion | Location (ROI or Bounding Box) | Number : 1 of 10 |
| Abnormality 2 | Malformation | Annotation | Number: 1 of 10 |
| Abnormality 3 | Metastasis | Location | |
| Comment | Resolved Lesion | Reference to a previous examination | |
| Measurement 1 | 3 cm | Associated text | Number : 1 of 10 |

**TABLE 4: Data relating to the report**

| **Type of data** | **Example of Possible Values** | **Example of Associated Element** |
|---|---|---|
| Pathology | Fracture, cancer, aneurysm | Location (ROI or Bounding Box) |
| Type of Examination | Abdomen, Thorax | |
| Type of Series | MRI, CT | MRI Sequence, Injection |

**TABLE 5: Data relating to the patient**

| **Type of data** | **Example of possible values** | **Example of associated element** |
|---|---|---|
| Gender | Male, Female | |
| Age | 12 Years Old | |
| Known Pathology | Cancer | Reference to a previous examination |
| Previous Examination | Fracture | Reference to a previous examination |

Creation of an IVIR according to the invention can be considered as a sequence of steps, as illustrated by **FIG.15****:**
- creating structured data by means of independent and specialized modules,
- merging independent or correlated data,
- determining the structure of the IVIR which corresponds to the protocoled examination,
- automatically generating additional post-processing data if necessary,
- associating data with the IVIR structure.

It is important to note that this presentation of these steps has been detailed as sequential only for the purpose to provide a clear explanation. Indeed, said steps could be achieved in a different sequence, iteratively. Some steps may be simultaneous or parallelized, with each providing the information required for another step.

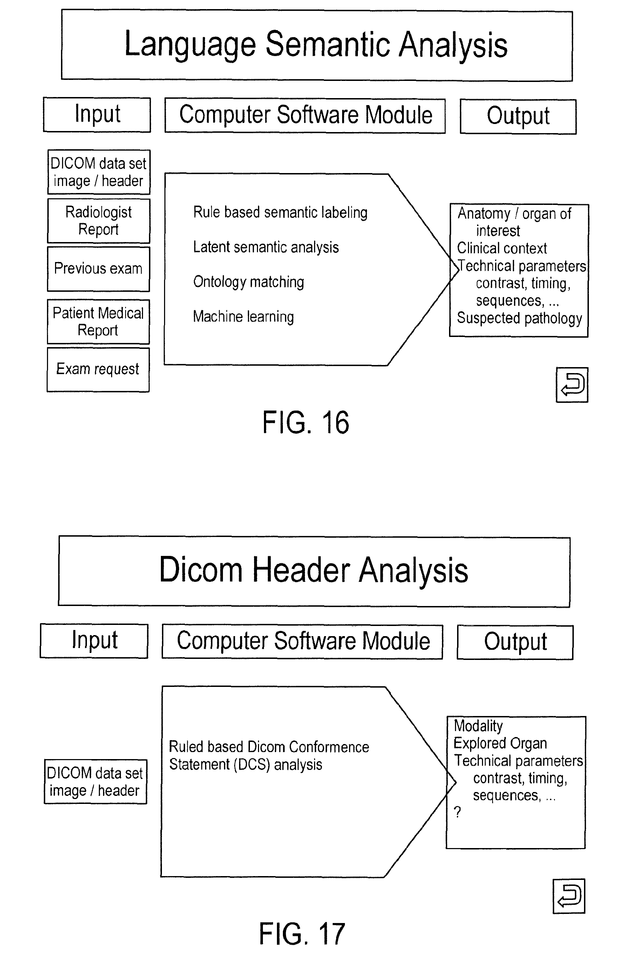

Structured data can be created by means of independent and dedicated modules. Initial processing would first consist of structuring contextual data available as inputs and as well as information collected from patient clinical course. To accomplish this, artificial-intelligence expert system modules may be necessary to process data inputs and generate structured semantic data. These dedicated system modules may include, as illustrated by **FIG. 16-19****:**
- a module for free-text or language semantic analysis,
- a module for analyzing DICOM headers,
- a module for image analysis and recognition,
- a module for analyzing the radiologist's behavior during the interpretation of imaging data.

With reference to **FIG.16****,** the language semantic analysis module comprises operations such as rule-based semantic labeling, latent semantic analysis, ontology matching and machine learning.

The DICOM header analysis module, illustrated by **FIG.17****,** implements a rules-based DICOM Conformance Statement (DCS) analysis.

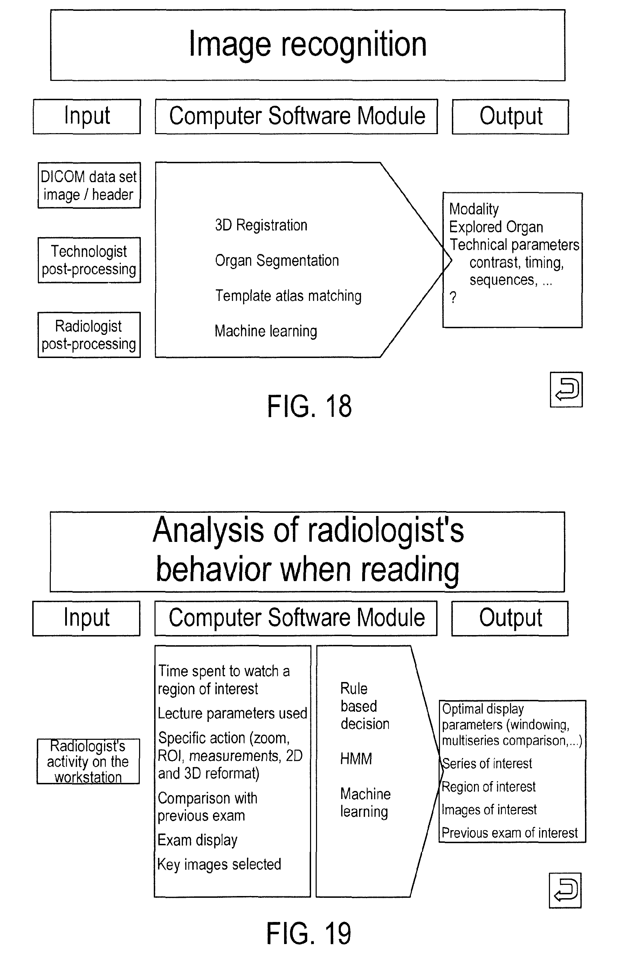

The Image Analysis and Recognition module (see **FIG.18****)** includes the operations of 3D co-registration, organ segmentation, template atlas matching and computer vision machine learning.

With reference to **FIG.19****,** the module for analyzing the radiologist's behavior during image interpretation implements rule-based decisions, Hidden Markov Models (HMM) and machine learning to process data including but not exclusive to:
- time spent viewing a region of interest (ROI),
- reading parameters used,
- specific actions (e.g. zoom, ROI, measurements, 2D and 3D reformats),
- utilization of comparisons with previous exams,
- examination displays,
- key images selected.

The outputs of this module consist of:
- optimal display parameters (windowing, multi-series comparison),
- series of interest,
- regions of interest,
- images of interest,
- previous examinations of interest.

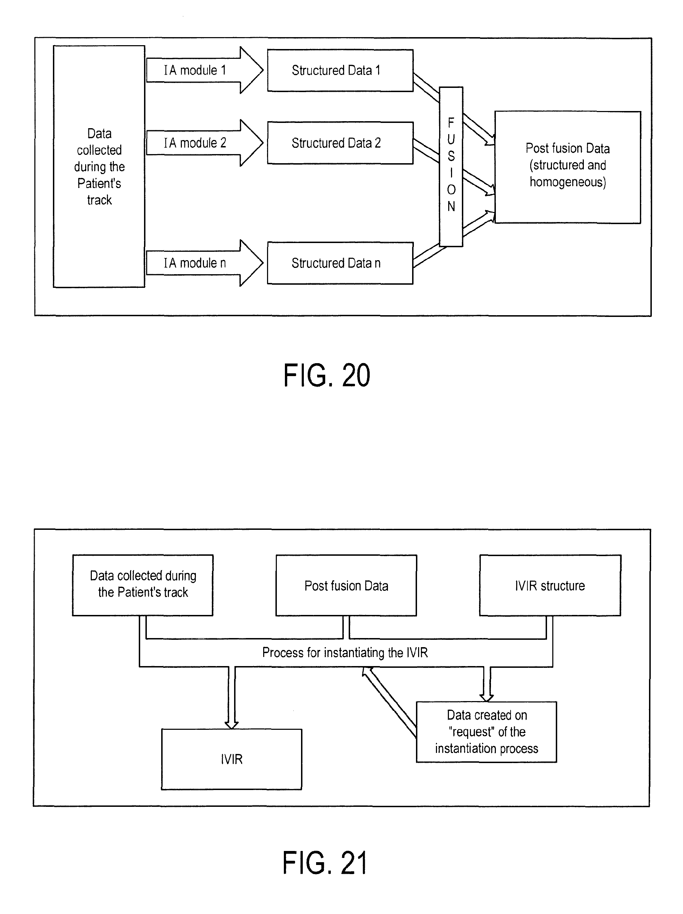

Data generated by the above-described modules are merged to generate structured and homogeneous data, as illustrated by **FIG.20****.**

This merging step is required for gathering redundant structured data, computing average values to detect abnormalities and localizing said abnormalities, and deciding when some contextual data should be preserved or deemed irrelevant and suppressed.

The merging step may either use deterministic rule-based algorithms or be derived from learning techniques on databases.

The result of the merging step is a set of structured and homogeneous data with a structure which is common for the overall IVIR building methods. These output data are called post-fusion data.

The data structure for an IVIR to be generated can be obtained via a deterministic algorithm applied to post-fusion data. However, said data structures can also be generated using a learning-based classifier algorithm. This is possible because many examinations have associated IVIRs which have similar structures and may constitute a natural grouping or family of IVIRs.

The classifier is designed to associate the post-fusion data with the structured template of the IVIR, and thus requires a learning database, a library of objects that may be attached to it, and for similar classes of objects (e.g. lesions), the number of mandatory descriptive objects that have to be attached, the level-of-detail to which it belongs, and for each type of object, a link to a node of the structure if said type of object should permit a node change when an action is performed by the user on this object.

Thus, with each node, it is possible to associate a screen of the IVIR, as the type of object to be displayed and the actions associated when said objects are known.

In the case where the object to be associated with a node of the template of the IVIR is part of the available information, instances of the corresponding object have to be found among the overall information collected or produced during the patient's clinical course or using the post-fusion data, with reference to **FIG.21****.**

For each type of data, it is necessary to identify or compute amongst the structured information the object which is the best corresponding to said type.

This can be done:
- on the basis of rules,
- on the basis of a distance which is *a priori* defined between structured information,
- on the basis of a distance between the structured information, which is created by a learning technique. A learning database is then required.

Some kinds of object to be associated with the node are not part of the elements collected during the patient's clinical course. For example, these may include some 3D rendering types or specific measurements. In such cases, they are automatically computed when needed.

Some types of rendering suited for various display formats and mobile supports (browsers, tablets, smartphones, etc.) can be implemented by means of IVIRs according to the invention.

Such mobile support may display standard data but be unable to handle volume or 3D rendering. Data that can be displayed is usually 2D images or 3D surfaces (i.e. polygonal surfaces). Thus, it is important to generate IVIRs that can be displayed on such mobile devices.

The "flyover" technique allows compatibility of any 3D display representation with standard browsers and mobile devices. This technique consists of pre-calculating a sample of all the views of the 3D object by moving a camera on a sphere covering said object to be displayed, and pointing the camera towards the sphere's center. Longitudinal and latitudinal data is associated with each 2D image constituting a view. Through said pre-calculated 2D images, it is possible to use a viewer having only standard display and pointing functionalities. This provides the user with the impression of manipulating a 3D object.

Now it will be described how to implement a learning database for the method according to the invention when necessary. A corresponding IVIR is created from information collected along the patient's clinical history. This implies generating intermediate data and using learning-based algorithms via:
- modules associating structured data with said collected information,
- a module for merging structured data produced by the independent modules into homogeneous and structured data,
- a module defining the IVIR structure from said collected information and from post-merging data,
modules associating the requested objects with the nodes of the template,
- modules automatically creating objects to be associated with nodes of the IVIR when requested.

When these modules are machine learning-based, they require a manually built database, the structure of which is simply arranged as a quadruplet set comprising:
- collected information
- post-merging data
- IVIR class
- the IVIR

A corresponding template with node-object association rules is associated to each IVIR class. This first database is built through an incremental process comprising the following steps:
- considering an examination, and collecting non-structured information associated with the patient's clinical history,
- assessing whether an IVIR structure corresponds to said examination,
- if so:
   - manually creating the corresponding IVIR and post-merging data,
   - arranging the quadruplet into the IVIR class corresponding to said structure,
- if not:
   - Creating a new IVIR structure and a new IVIR class.

When this first database is available, it must be ensured that, to maximize the success of the IVIR generation, all collected information is available as an input, the learning-based algorithms are able to produce the post-merging data, the system is able to identify the IVIR class and ultimately create the IVIR. If any of these steps are not possible, the structure of intermediate data should be modified.

The scope of the present invention is of course not limited to the above proposed mechanisms and other technical options can be considered for implementing the invention.

## Claims

1. A method for automatically generating a mode of presentation for data collected and produced during an imaging examination for which an examination report is done by an operator in charge of said examination, comprising:
- analyzing contextual information related to said examination,
- analyzing data contained in said examination report,
- eliciting and producing relevant information from and within said collected and produced data, based on results of said contextual information analysis and of said report data analysis, and
- displaying said relevant information, in a simplified multi-dimensional manner as an interactive visual imaging report (IVIR), wherein said contextual information comprise information on the behavior of said operator while achieving and reporting said examination.

2. The method according to Claim 1, wherein the information on the operator's behavior include information among the following set of information: time spent watching specific images or series of images, choice of the parameters used to review the images, zooming on a specific area of interest, observing a point of interest, selecting/deselecting an image, observing/creating a region of interest or using measurement tools, choosing a key-image, comparing the examination with others set of images previously acquired, using post processing tools on an image or a series of images.

3. The method according to any of preceding Claims, **characterized in that** the contextual information comprise written information contain in the imaging examination ordering

4. The method according to any of preceding Claims, **characterized in that** the contextual information comprise information extracted from a patient's electronic medical record..

5. The method according to any of preceding Claims, **characterized in that** the contextual information comprises information on the process of creation of the examination.

6. The method according to Claim 5, **characterized in that** the contextual information on the process comprises information on the protocol chosen for producing the examination.

7. The method according to any of preceding Claims, wherein relevant information is displayed as a multi-dimensional schematic rendering.

8. The method according to Claim 7, wherein the multi-dimensional schematic rendering provides with a multi-scale functionality with a plurality of interactive visual imaging report(IVIR) levels, said multi-dimensional schematic rendering containing objects which can be clicked so that the IVIR level is changed.

9. The method according to Claim 8, wherein a IVIR level contains a plurality of displays.

10. The method according to Claims 7 to 9, **characterized in that** the multi-dimensional schematic rendering comprises a tridimensional rendering that can be displayed with a low computing weight.

11. The method according to any of Claims 7 to 10, **characterized in that** the multidimensional schematic rendering comprises a 2D or 3D rendering of relevant information that is superimposed on reference organs.

12. The method according to any of Claims 7 to 11, **characterized in that** the multidimensional schematic rendering comprises a highlighted display of relevant information on one or more multidimensional anatomic objects of interest within said rendering.

13. The method according to any of Claims 7 to 12, **characterized in that** the multidimensional schematic rendering comprises displaying information that are acquired or produced during the examination, said displayed information being superimposed on a multidimensional anatomic object in relation of said information.

14. The method according to any of Claims 7 to 13, **characterized in that** a process for multidimensional schematic rendering is determined from the analysis of contextual information.

15. The method according to any of Claims 7 to 14, **characterized in that** a mode for displaying the multidimensional schematic rendering is determined from the analysis of contextual information.

16. The method according to any of preceding Claims, further comprising a sequence for creating an interactive visual imaging report (IVIR) including following steps:
- creating structured data from information collected during a patient's prescribed examination track,
- merging independent or correlated data,
- determining a structure for said IVIR, which corresponds to said prescribed examination,
- automatically creating data if necessary, and
- associating said structured or created data with said IVIR structure.

17. The method according to Claim 16, implementing artificial intelligence tools for processing input data and generating structured data, said input data including contextual information.

18. The method according to any of Claims 16 to 17, further implementing tools for free-text or language semantic analysis.

19. The method according to any of Claims 16 to 18, further implementing tools for analyzing DICOM headers in the field of medical imaging.

20. The method according to any of Claims 15 to 19, further comprising tools for image analysis and recognition.

21. The method according to any of Claims 16 to 20, further comprising tools for analyzing the operator's and reader's work during producing and interpreting the image dataset , in view to produce contextual information on said operator's behavior.

22. The method according to any of Claims 16 to 21, wherein the IVIR data structure is obtained by means of a determinist algorithm applied to input data and/or post-merging data and/or contextual data.

23. The method according to any of Claims 16 to 22, wherein the IVIR data structure is generated using a learning-based classifier algorithm.

24. The method according to any of Claims 16 to 23, further implementing a learning data base arranged as sets of quadruplets including each comprising collected information, post-merging data, an IVIR family and an IVIR.

25. The method according to Claim 24, wherein a corresponding template with node-object association rules is associated with each IVIR family.

26. The method according to any of Claims 24 or 25, wherein said learning data base is built through an incremental process.

27. An interactive visual imaging report, in relation with a medical examination achieved on a patient, comprising:
- a global multidimensional view of said patient, including graphic information pointing one or multiple organ of interest concerned by said examination, and
- a superimposed multidimensional view that highlight the findings detected during the process of reporting the examination.

28. The interactive visual imaging report of Claim 27, further comprising a plurality of interactive visual imaging report (IVIR) levels for displaying images, data extracted from images or schematics of the organ of interest, said interactive visual imaging report being provided with a multi-scale functionality for moving from one of said IVIR levels to another IVIR level.

29. The interactive visual imaging report of Claims 27 or 28, further comprising a multidimensional rendering of the organ of interest, said multidimensional rendering being accessed by rotating and/or translating and/or zooming on said organ.
